# EUROPEAN PATENT APPLICATION

(11) **EP 4 023 214 A1**
(43) Date of publication of application: **06.07.2022**
(21) Application number: 21150238.0
(22) Date of filing: 05.01.2021
(51) Int. Cl.: A61K 9/16, A61K 9/50, A61K 9/06, A61K 47/36, A61K 31/122, A61K 31/155, A61K 31/355, A61K 31/375, A61K 31/592, A61K 31/593, A61K 33/04, A61K 33/30, A61P 1/02, A61P 11/02, A61P 11/04, A61P 17/02, A61P 31/02, A61P 31/04, A61P 31/10

(54) **SLOW-RELEASE DEVICE FOR WOUND DISINFECTION AND ITS USAGE**

(71) Applicant: Justus-Liebig-Universität Gießen, 35390 Gießen (DE)
(72) Inventor: Meyle, Jörg, 35444 Biebertal (DE); Gröger, Sabine, 35390 Gießen (DE)
(74) Representative: Stumpf, Peter

(57) **Abstract**

The invention is about a device, for example a microsphere, constructed for the slow release of pharmaceutically active substances and its use for disinfection of wounds/niches/periodontal pockets.

## Description

The invention relates to a device constructed, respectively composed, for the slow release of pharmaceutically active substances (synonymously also called **A**ctive **P**harmaceutical **I**ngredients (**API**s) or pharmaceutically active compounds), the production of that device and its use for wound disinfection, e.g. pocket disinfection. The expression "device" - as it is used herein - comprises polyhexamethylene biguanide (PHMB) containing paraffin-particles, particles, microparticles, microgranules, microspheres with core-shell-structure or without core-shell structure.

### Field of the invention

The general field of the invention is the area of disinfection and the therapy of acute or chronic inflammation. The core-field of the invention is wound-disinfection, especially the disinfection of niches and (periodontal) pockets in the area of medical treatment.

### Background of the technology

According to the state of the art, the therapy of acute or chronic inflammations is trying to stop the pathogenic processes causing the illness and to support the healing processes by removal of the pathogens.
In order to do so, in the past so-called "slow-release devices" (e.g. non-inventive microcapsules, non-inventive granula, non-inventive micro-particles, non-inventive nano-particles) have been developed, which release pharmaceutically active compounds/substances, resp. active pharmaceutical ingredients (API's) - all these terms are used herein synonymously - over a period of time in order to stop the inflammatory process.

According to the state of the art only disinfectants like chlorhexidine and other molecules with antiseptic activity and antibiotics (metronidazole, minocycline, penicillines, tetracyclines) are applied.

Due to pathophysiological processes in the area of inflammation cytokines and proinflammatoric signal-molecules are released, which are loosening up the tissue and support the infiltration by immune cells. Immigrating leucocytes release upon activation and during phagocytosis oxidizing substances (e. g. reactive oxygen species = ROS) which inactivate and kill the pathogens, but are causing at the same time additional damage to the inflamed tissue.

Polyhexanide (polyhexamethylene biguanide, PHMB) is a polymeric substance broadly used as disinfectant and antiseptic suitable for clinical use in critically colonized or infected acute and chronic wounds. Its beneficial characteristics are particularly attributable to its broad antimicrobial spectrum, good cell and tissue tolerance, ability to bind to the organic matrix, low risk of contact sensitization, and wound healing promoting effect. In addition, no development of microbial resistance during PHMB use has been detected to date, nor does this risk appear imminent. The aim of therapy using PHMB is to reduce the pathogen burden in a critically colonized or infected acute or chronic wound.

PHMB is a strong base and interacts with acidic, negatively charged phospholipids in the bacterial membrane that leads to increased fluidity, permeability and loss of integrity, followed by the death of the bacteria. Due to its nonspecific, strong interaction with negatively charged phospholipids, PHMB has a broad antimicrobial spectrum, including Gram-positive and Gram-negative bacteria, plaque-forming and biofilm-building bacteria, spore-forming bacteria (but not bacterial spores), and intracellular bacteria such as *Staphylococcus aureus, Bacillus subtilis, Streptococcus faecalis, Streptococcus lactis, Escherichia coli, Enterobacter cloacae, Pseudomonas aeruginosa, Saccharomyces cerevisiae,* chlamydiae and mycoplasma, and fungi including *Candida* spp. as well as *Aspergillus* spp. PHMB is classified as 'practically nontoxic', based on the low oral toxicity of 5 g/kg in rats. PHMB showed neither sensitizing nor photosensitizing effects in animal tests. In contrast to chlorhexidine that is regularly reported to lead to late-onset hypersensitivity, eczema and even to severe anaphylactic reactions, PHMB seems to carry only a slight allergic risk and remains an uncommon contact allergen. *In vitro* results from cell culture tests and explant tests show that PHMB has remarkably low cytotoxicity as shown in murine fibroblasts, rat heart tissue and fetal rat humeri as well as tests on wound healing in a guinea pig model.

In conclusion the outstanding relation of PHMB between antimicrobial efficacy and low cytotoxicity and its exceptional tissue compatibility makes it a promising anti-infective substance with highly interesting features.

Chlorhexidine (CHX) is a long known antiseptic substance that is routinely used for the treatment of gingivitis and periodontitis. It has been considered as gold standard in chemical control or oral bacterial biofilms and periodontal anti-infective therapy. Referring to the manufacturer PerioChip^{®} is a biodegradable chip, which contains 2.5 mg of chlorhexidine gluconate. PerioChip^{®}, when combined with conservative periodontal treatment, e.g. scaling and root planing (SRP) procedures, has been shown to reduce pocket depth (PD) in patients with adult periodontitis and is recommended for use in periodontal pockets with a PD greater than or equal to 5 mm. It is a thin wafer that is inserted under the gums in those areas where pockets are 5 mm or deeper. Because of the biodegradable gelatin matrix it dissolves with no need of removal. It has been shown, *in vitro,* to fight bacteria for a period of seven to ten days. In a randomized clinical and microbiological trial including 15 patients diagnosed with chronic periodontitis analyzing the major periodontopathogenic bacteria *Porphyromonas gingivalis, Prevotella intermedia, Aggregatibacter actinomycetemcomitans,* and *Fusobacterium nucleatum* a significant improvement in all clinical variables in the test group as compared to the control group over the study period was demonstrated. Total colony counts were significantly reduced in the test group as compared to control over the period of time.

CHX is increasingly being used not only as an antiseptic to prevent hospital infections and an adjuvant in oral hygiene but also as a preservative in personal care products. Following more widespread exposure to the agent, reports of adverse reactions are increasing. Complications can range from mild irritant contact dermatitis to life-threatening anaphylaxis. In some cases allergic contact dermatitis preceded anaphylaxis.

Hydrolyzed gelatin is of animal origin (porcine, bovine) and besides the risk of allergic reactions against foreign proteins gelatin is not suitable for a vegetarian alimentation and because of the porcine fraction it may be refused by some persons due to religious belief.

CHX also has major disadvantages, for example it exerts an anti-proliferative effect on fibroblasts, causes inhibition of adhesion of fibroblasts to the root surface and suffers a rapid inactivation by serum proteins.

These are severe disadvantages of the state of the art. Another major disadvantage of the state of the art is that there are no devices (non-inventive micro granules, non-inventive microspheres, non-inventive micro particles etc.) existing which exert long time efficacy beyond about two to four weeks.

Further methods/devices for supporting the therapy of acute or chronic inflammations beyond just trying to stop the processes causing the illness and supporting the healing-processes by removal of the pathogens, i. e. by providing disinfection, are also unknown to date.

### Content of the invention

An objective of the present invention is to provide a device (preparation, microsphere etc.) constructed, resp. composed, for the slow release of at least one pharmaceutically active substance (i. e. "active component", "drug", "active pharmaceutical ingredient"), allowing the long-term release for at least two or more weeks of therapeutically effective amounts of the at least one pharmaceutically active substance. Another objective of the present invention is the production of that device and its use for pocket disinfection. The device according to the invention preferentially comprises PHMB as pharmaceutically active substance/compound/API, but may also additionally or instead of PHB comprise other disinfectants as pharmaceutical effective substances. The device comprises particles containing the active components and is herein also synonymously referred to as "paraffin granules", "paraffin particles", PHMB paraffin-particles, "particles" "microspheres", "microparticles", "micro-granular compound", "preparation".

It is well known to a person of ordinary skill according to the state of the art that the specification of a range of values always also includes any single value within that range, even if not explicitly mentioned. Therefore, any range of values, parameters or any other numerical values given herein also includes all values in between, e.g. a range from 2 to 8 also includes the values 3, 4, 5, 6 and 7. A person of ordinary skill will also recognize that additional ranges of concentrations and primary or secondary particle sizes within the explicit ranges mentioned herein are contemplated and are within the present disclosure. The same is obviously valid for ranges of time mentioned herein.

In order to achieve the above objective, the present invention provides new types of PHMB containing microspheres prepared of different materials. The kinetics of the active component (API) release is analyzed as well as the anti-bacterial efficacy.

The words "substance" and "compound" as well as all grammatical forms thereof are used herein synonymously. The same is true for the phrases "microsphere", "micro particle", "paraffin particle", "microgranula", "beads", particle and "granula".

The invention is applicable for the prevention and/or therapy of wounds and/or acute or chronical inflammations on mammals, so called niche-inflammations. The invention is therefore applicable for humans and/or animals.

The antibacterial efficacy of PHMB stay detectable over the whole time period of observation (56d). While not wanted to be limited by theory, the particles probably melt after 24h stirring in 37°C and form an emulsion, whereby the process of melting supports/enhances the release of the at least one pharmaceutical active substance contained within them. Nevertheless the melting process does not cause the melted particles to coagulate which is proven by the fact that an emulsion is formed. Thus the microscopic pictures of the "particles" acquired later than 24 h of the beginning of the experiment, indeed are not necessarily pictures of the particles themselves but - at least in some cases - pictures of melted particles, i.e. drops of the oily phase of the emulsion. Therefore it is comprised by the scope of the invention to vary the type of paraffin used for producing the particles in order to adjust this melting process and thereby the release kinetics of the at least one pharmaceutical active substance. The PHMB is (exemplarily) released from the beads in the following kinetics: After the first 24h 1/5 of the calculated PHMB concentration (10 µg/µl) is detected (4.027 µg in 2 µl). The concentrations that are exemplarily quantified in the samples from 48h, 4d, 8d ,16d, 28d and 32d are 86%, 79%, 74%, 63%, 49% and 45% of the 24h value (3.456 µg, 3.161 µg, 2.961 µg, 2.538 µg, 1.970 µg and 1.806 µg in 2 µl respectively). The presence of serum seems to inhibit the release or the detection of the PHMB.

The method for measuring the antiseptic activity of the particles/devices is performed in vitro by admixing the particles/devices together with the test organisms (bacteria) into the culture medium, e.g. agar plates or gelatin plates, and observing the growth of the bacteria. Around the particles the growth of the bacteria is visibly inhibited, forming so called "inhibition zones"/"growth inhibition zones" around the particles where no bacteria are growing. The size of these inhibition zones is the measure of the activity of the particles. How the size of these inhibition zones is measured is marked up within the figures. The measured raw values may be used or mean values may be calculated for evaluation.

The size of the growth inhibition zones from the maximum values reached until the end of the time of observation (usually about 32-56 days) is reduced only to about 50 % of the maximum value, indicating the effectiveness of the particles/devices/preparations according to the invention well beyond the state of the art. The experimental findings well allow to extrapolate the effectiveness beyond the time of observance (56 days) until at least 60 days.

It is known from previous clinical studies that the healing period after mechanical periodontal pocket treatment by scaling and root planning (SRP) requires a period of at least 3 months. After 6 months radiographic changes and new bone formation can be observed, thus it is of major interest to develop and use a device which will avoid early recolonization of the treated area, which could hamper the healing outcome.

A matrix material, e.g. glycerin, water or anything else can be a component of the core of the microsphere and/or a component of the shell of the microsphere.

The developed micro granulates allow - due to their small size - the application in infected resp. inflamed niches and fissures as, for example, gingival/periodontal pockets, fistula tracts and abscess holes of various dimensions.

The invention comprises microgranula/preparations which, additionally to the release of the at least one API, do - at the same time, before or after the release of the at least one API - also release at least one compound with anti-inflammatory or otherwise healing supportive, efficacy.

Thus the invention can be summarized as follows:
The invention comprises a long-acting (at least for 60 days, according to extrapolation from experimental data (56 days) event 70 - 80 days) microsphere comprising polyhexamethylenebiguanidehydrochloride or its salt as a drug and at least one matrix material, which is independently from each other chosen from the list of matrix materials comprising paraffin wax, glycerin-monostearate, water, lecithin, glycerin, alginate. The microsphere comprises polyhexamethylenebiguanidehydrochloride or its salt and an amount of paraffin wax per percent by weight of the sum of the components from 40 % by weight to 70 % by weight and/or an amount of glycerin-monostearate per percent by weight of the sum of the components from 5 % by weight to 30 % by weight and/or an amount of water per percent by weight of the sum of the components from 50 % by weight to 30 % by weight and/or an amount of lecithin per percent by weight of the sum of the components from 0,5 % by weight to 20 % by weight. The microsphere also comprises an amount of glycerin per percent by weight of the sum of the components which is within the range from 50 % by weight to 90 % by weight, an amount of alginate per percent by weight of the sum of the components which is within the range from 30 % by weight to 50 % by weight. The microsphere has an average particle size of about 10 to 500 µm and is satisfying at least one of the following two conditions:
a) The remaining ratio of the drug after 8 days in a release test is at least 70 % of the concentration detected after 1 day in the release test and/or
b) The remaining ratio of the drug after 32 days in a release test is at least 40 % of the concentration detected after 1 day in the release test.

The microsphere is either core-shell-structured or exerts a basically uniform structure. If the microsphere is core-shell-structured it exerts the following characteristics: The core of the microsphere comprises polyhexamethylenebiguanidehydrochloride or its salt and an amount of paraffin wax per percent by weight of the sum of the components of the core from 50 % by weight to 60 % by weight and/or an amount of glycerin-monostearate per percent by weight of the sum of the components of the core from 10 % by weight to 20 % by weight and/or an amount of water per percent by weight of the sum of the components of the core from 10 % by weight to 20 % by weight and/or an amount of lecithin per percent by weight of the sum of the components of the core from 1 % by weight to 10 % by weight. The shell of the microsphere comprises an amount of glycerin per percent by weight of the sum of the components of the shell which is within the range from 60 % by weight to 80 % by weight and an amount of alginate per percent by weight of the sum of the components of the shell which is within the range from 20 % by weight to 40 % by weight.

The microsphere may further comprise at least one pharmaceutically active substance, independently from each other chosen from the list comprising sodium ascorbate, selenium, zinc, vitamin D, vitamin E, vitamin K, vitamin K2, coenzyme Q10.

The microsphere may further comprise at least one pharmaceutically active substance, independently from each other chosen from the list comprising sodium ascorbate, selenium, zinc, vitamin D, vitamin E, vitamin K, vitamin K2, coenzyme Q10, wherein the at least one more pharmaceutically active substance is located either in the core and the shell of the microsphere or only in the core of the microsphere or only in the shell of the microsphere if the microsphere is core-shell-structured.

The microsphere may have an average size of no more than 300 µm.

The invention also comprises an agent comprising the microsphere of any one of the types described before or a multitude of microspheres as described before for use in preventing and/or treating wounds and/or acute or chronical inflammations on mammals,

The agent as described above can additionally be characterized in that the microsphere or the multitude of microspheres is embedded in a gel comprising hyaluronic acid within the range from 0.001 % by weight to 10 % by weight and sterile water, whereat the hyaluronic acid comprises hyaluronic acid molecules with a molecular weight below 403 kDa.

The agent according to any type as is described above, can additionally be characterised in that the agent is sterile, whereat the sterility is achieved by applying heat, uv-radiation or addition of an antimicrobial compound to the agent during manufacturing. The person skilled in the art well knows how sterilization is performed during manufacturing of the agent.

The agent according to any type as is described above can be used in preventing and/or treating wounds and/or infections in mammals, being located at abscess-cavities and/or fistula cavities and/or gingival cavities and/or peri-implant niches.

The agent according to any type as is described above can be used in preventing and/or treating peri-implant inflammation.

The agent according to any type as is described above can be used in preventing and/or treating hidden infections fistulae, fissures and in regions adjacent to the root of tongue or the tonsils.

The agent according to any type as is described above can be used in preventing and/or treating sinusitis.

The agent according to any type as is described above can be used in preventing and/or treating acute and/or chronic inflammations of niches of mucous membranes.

The agent according to any type as is described above can be used in preventing and/or treating acute and/or chronic inflammations of implant pockets.

### Detailed embodiments of the invention

### Materials and Methods

Polyhexanide (PHMB) containing micro particles
PHMB content: 10% by weight (exemplarily)
Carrier material: Paraffin (54 % by weight) (exemplarily)
Particle size: 200 µm ± 50 µm (exemplarily)

### Analysis of the release kinetics of the PHMB samples

For the investigation of the release kinetics of PHMB paraffin-particles, the PHMB paraffin-particles are weighted and the same weight of every sample is used for analysis.

The PHMB paraffin-particles are suspended in 5000 µl HBSS (Hanks' Balanced Salt solution) with 10 mM HEPEs buffer (N-(2-Hydroxyethyl)piperazine-N'-(2-ethanesulfonic acid)) and 50% fetal calf serum (FCS) without antibiotics in a sample tube with a lid. To obtain 50 mg (10 µg/µl) PHMB total concentration in the solution 500 mg is used. A magnetic stir bar is added and the tube is closed. The substances are incubated at 37°C, e.g. on a cable-free stirrer.

After 24h, 2d, 4d, 8d, 16d, 21d, 28d, 32d, 42d and 56d the samples are investigated visually and the grade of resolution is controlled and recorded.

A sample of 1000 µl particle free supernatant is harvested, the last one after complete resolution. An aliquot (800 µl) is frozen at -80°C for future analysis. The other aliquot is used to analyze the antibacterial properties. The volume removed is replaced after every sampling.

As negative control the same HBSS with FCS from the bottle prepared for the samples is frozen at -20°C and analyzed identically to the test samples.

All samples are prepared in duplicate in three different experiments.

### Analysis of the anti-bacterial properties of the PHMB samples

For the characterization of the anti-bacterial activity of the PHMB released from the test samples, the supernatants removed from the release kinetics experiment after 24h, 2d, 4d, 8d, 16d, 21d, 28d, 32d, 42d and 56d incubation with HBSS are used. As negative control HBSS with FCS is applied.

### Plate coating:

The blood agar plates are coated with - for example - *Streptococcus gordonii (S. gordonii)* cultured in brain-heart-infusion broth (BHI), 100 µl bacterial solution is distributed over the plates using a microbiological bail with 10 µl volume. In the agar plates gaps are punched out using a 5 mm diameter biopsy punch.

### Addition of the test solutions:

The PHMB containing solutions from every incubation time (40 µl) are placed in the gaps of 2 x 2 plates per solution. As negative control (one plate) 40 µl HBSS is used. The plates are incubated for 24 h at 37 °C.

### Measurement and documentation:

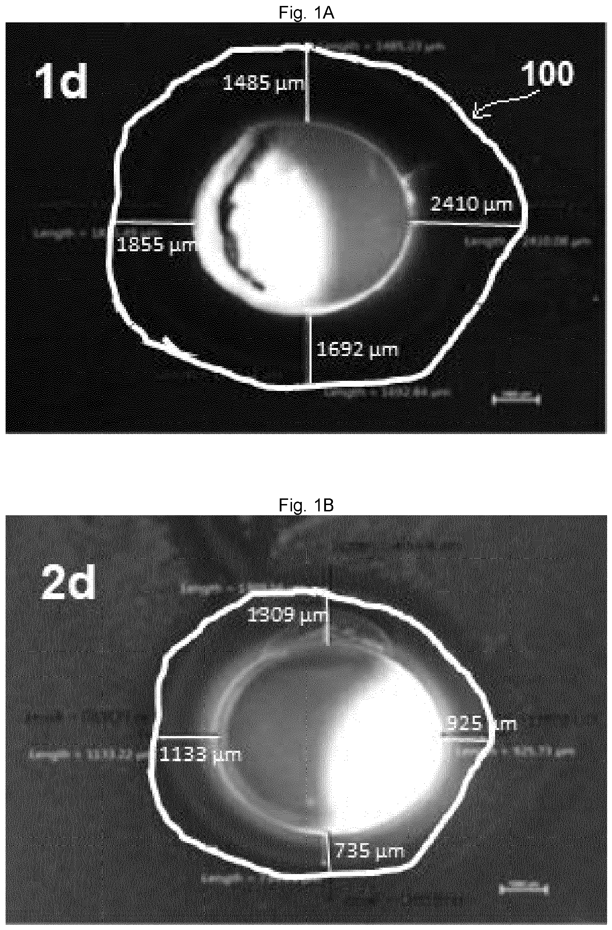

After the incubation time, the plates are visually analyzed respective to development of zones of bacterial growth inhibition. The plates are placed in a microscope with integrated unit of length measurement and camera and the inhibition zone is measured and photographed. The detected inhibition zones and the measured values of their size ("length", "width", resp. "distance"/"extension" from the surface of the particle) are exemplarily marked within Figs. 1A-G, 2A-C, 3A-D and 4A-C.

### Statistical analysis:

Statistical analysis is done according to the state of the art: The results are analyzed using independent two-sample Student's test. The character of the evaluation is explorative. The probability of error is set at 5% and is shown as p-values.

### Quantification by mass spectrometry:

**Table 1: Chromatographic conditions**

| Column | VDSpher Pur 100 SIL |
|---|---|
| Particle Size, Length × inner diameter | 4µm, 150x2.0 mm with precolumn |
| Order number | N1520E000VPI |
| Separation mode descriptions | analytical, normal phase |
| Mobile Phase | Solvent A: 15 mM ammonium formiate pH 3.5 |
| | Solvent B: acetonitrile |
| Elution conditions | 0 min 10% Solvent A |
| | 1 min 10% Solvent A |
| | 10 min 80% Solvent A |
| | 12 min 80% Solvent A |
| | 14 min 10% Solvent A |
| | 25 min 10% Solvent A |
| Flow rate | 300 µl/min |
| Injection | 20 µl |
| Column temperature | 30°C |
| Pressure | 70-160 bar |
| HPLC system | Alliance HT Waters 2790 with Waters micromass ZQ 4000 |
| | Detection: +ESI mass trace |
| | PHMB (dimer) m/z 367 |
| Sample and sample preparation | 10% Solvent A / 90% Solvent B |

### Analysis of melting of the PHMB particles

For the investigation of the melting behavior of PHMB paraffin-particles, the particles are prepared and incubated in the same manner as for the release kinetics. Supernatants are harvested directly after suspension, after 1d, 2d, 4d, 8d, 16d, 21d, 28d, 32d, 42 d and 56d. A volume of 100 µl is placed on a slide and analyzed and photographed in an invert microscope.

### Experimental Results

### Antibacterial properties:

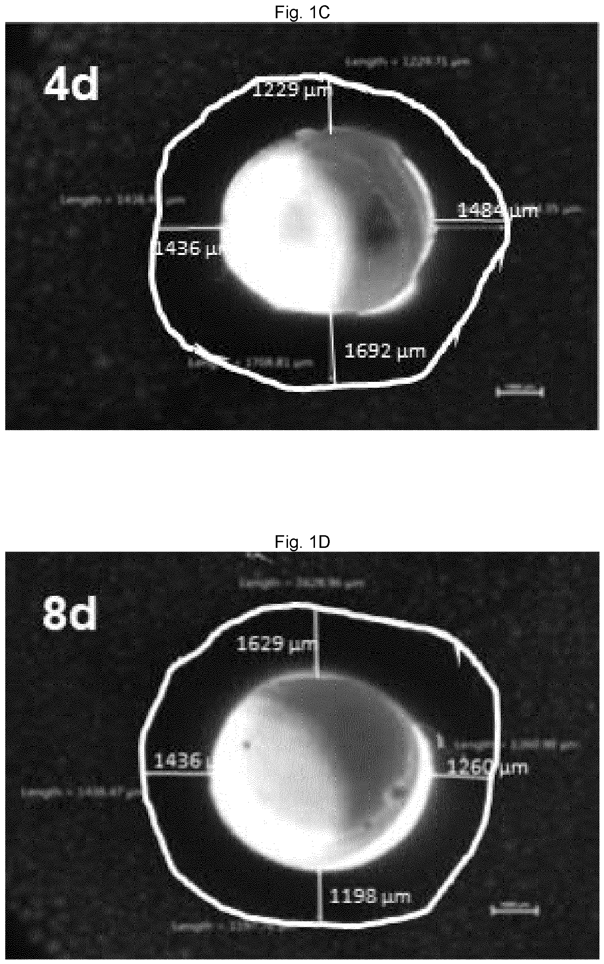

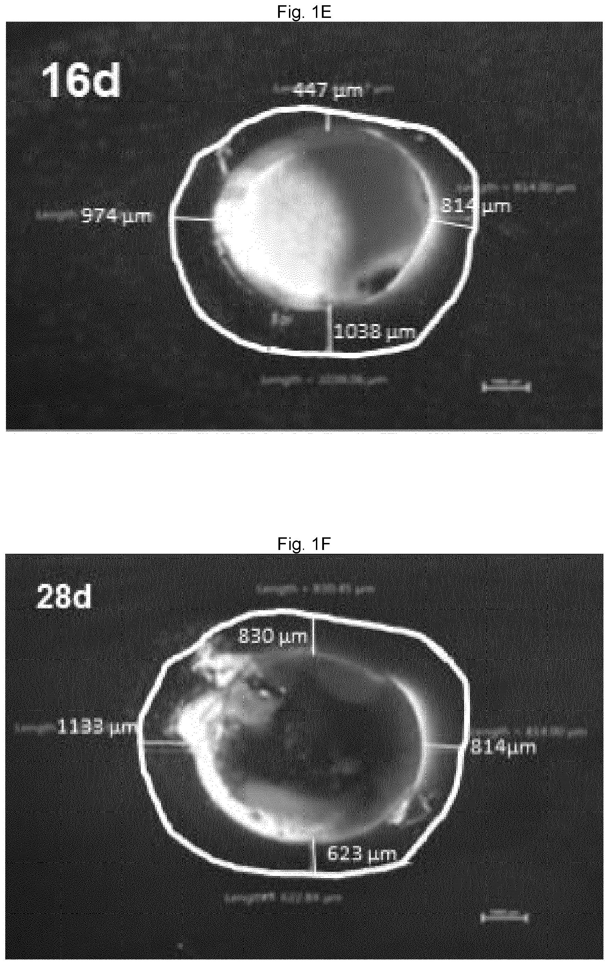

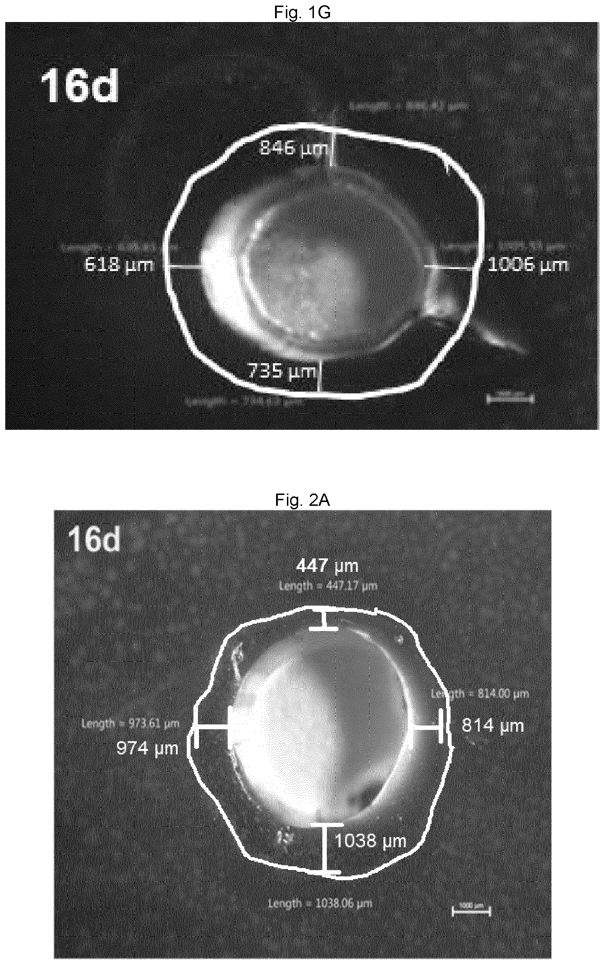

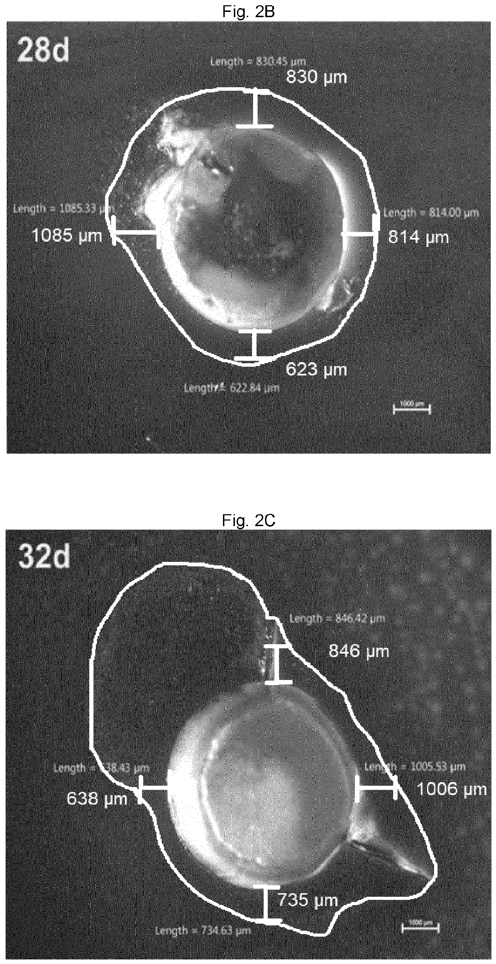

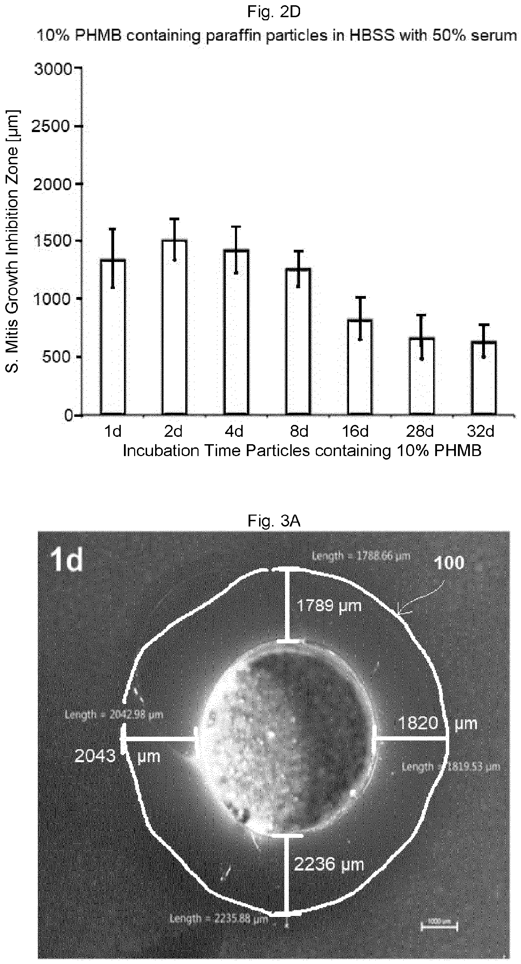

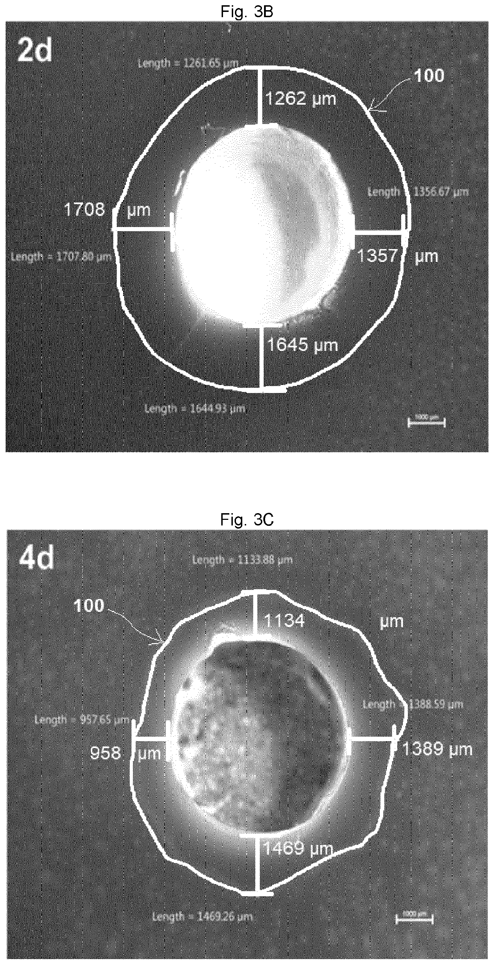

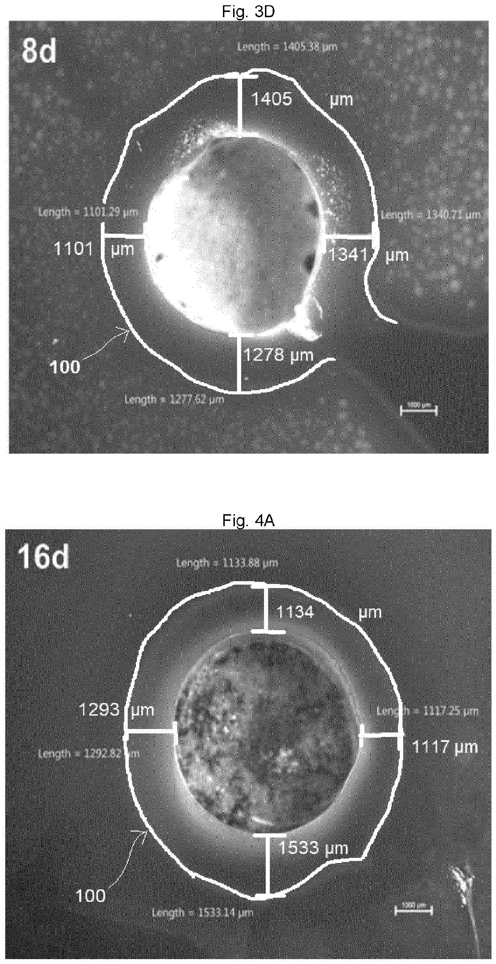

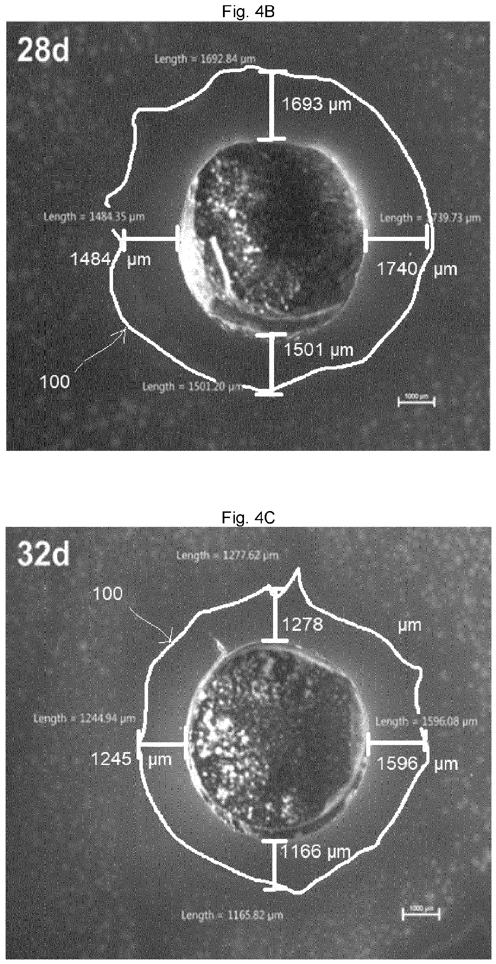

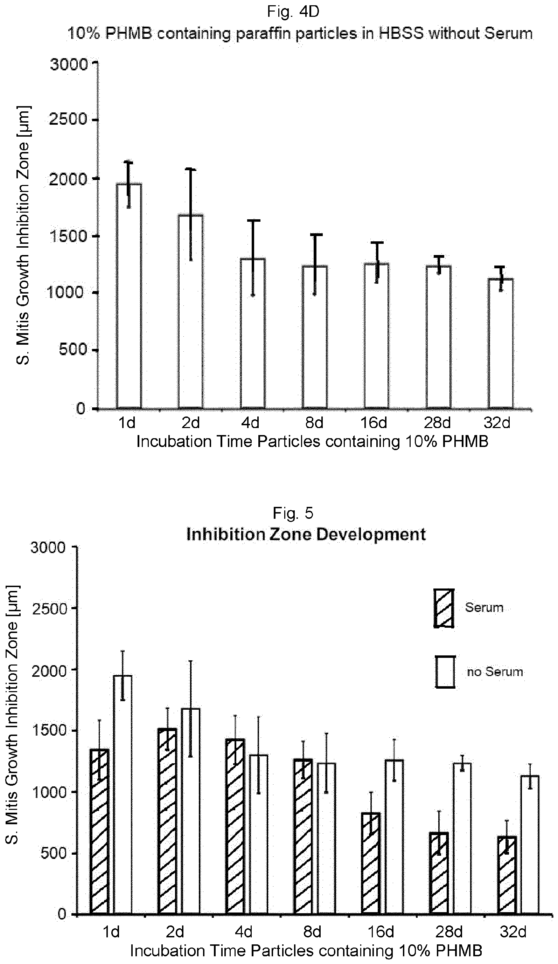

The mean inhibition zone of the growth of - for example - *S*. *gordonii,* i.e. in the presence of, paraffin particles (Fig. 1, 2) containing 10% PHMB in HBSS with 50% FCS developed over time from 1342 µm after 24h to 1512 µm, 1425 µm, 1262 µm, 825 µm, 665 µm to 633 µm after 2 days (d), 4d, 8d, 16d, (Fig. 1) and 28d, 32d (Fig. 2). The mean inhibition using HBSS without serum (Fig. 3, 4) altered from 1948 µm after 24h to 1678 µm, 1299 µm, 1235 µm, 1259 µm, 1235 µm and 1128 µm after 2 d, 4d, 8d, 16d, (Fig. 3) 28d and 32d (Fig. 4). In both cases the paraffin particles disappear after 24h (i. e. turn into drops of an emulsion, cf. above) and the solution appears turbid and milky. Fig. 5 shows the development of the inhibition zones with/without serum in comparison over time. The expressions "inhibition zone" and "inhibition" are used herein synonymously where applicable; the quantitative measurement of the inhibition zone is made by measuring at least four distances of the border line of the inhibition zone from the surface of the particle, resp. the location of the former surface of the particle after it has disappeared, by use of microscopic equipment as is known to the person skilled in the art.

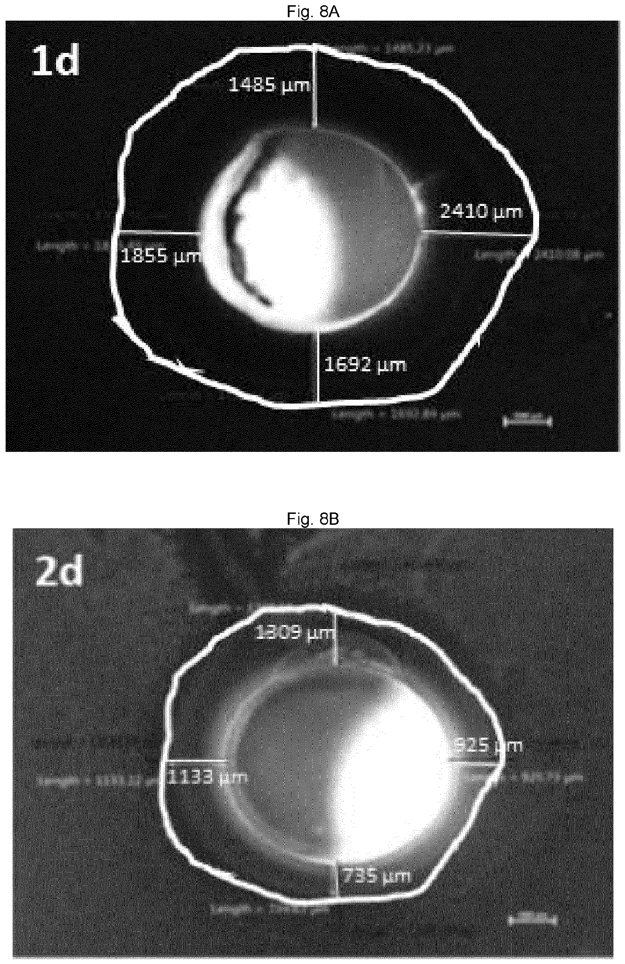

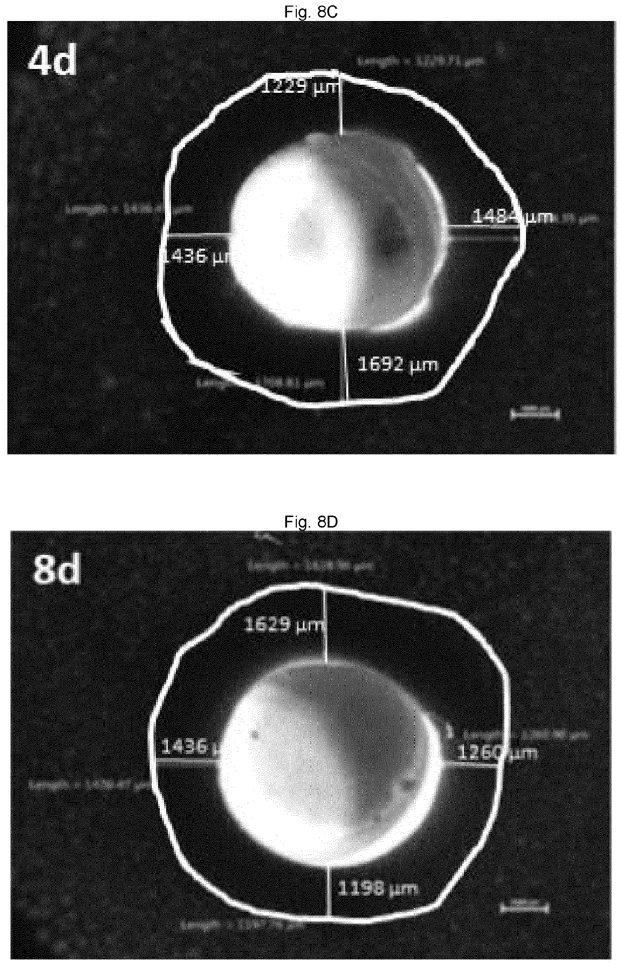

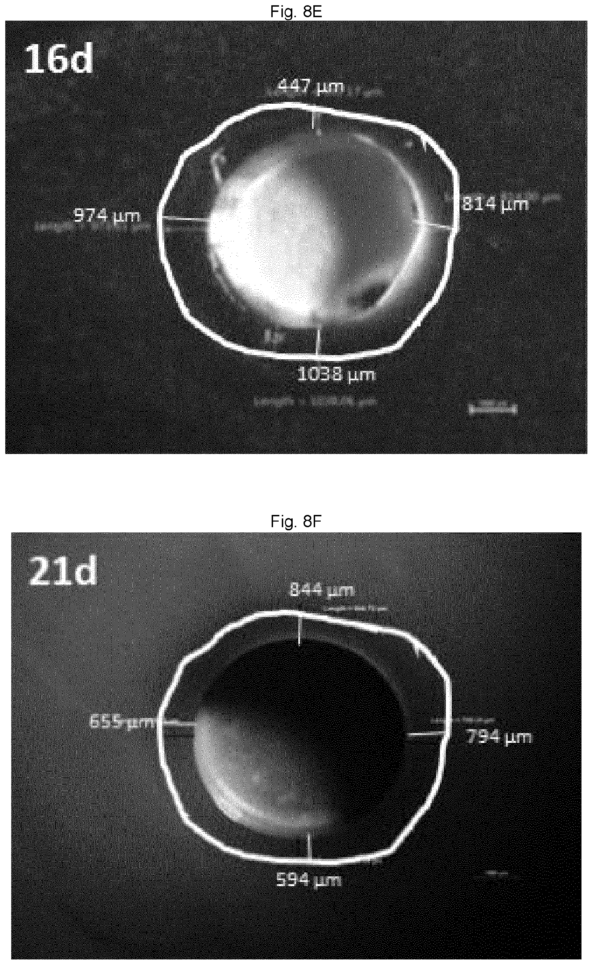

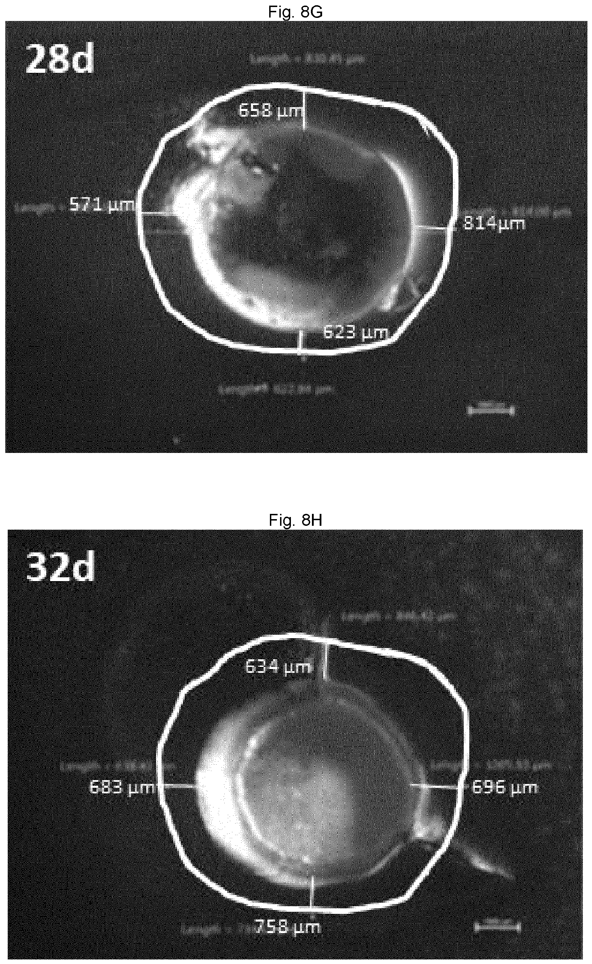

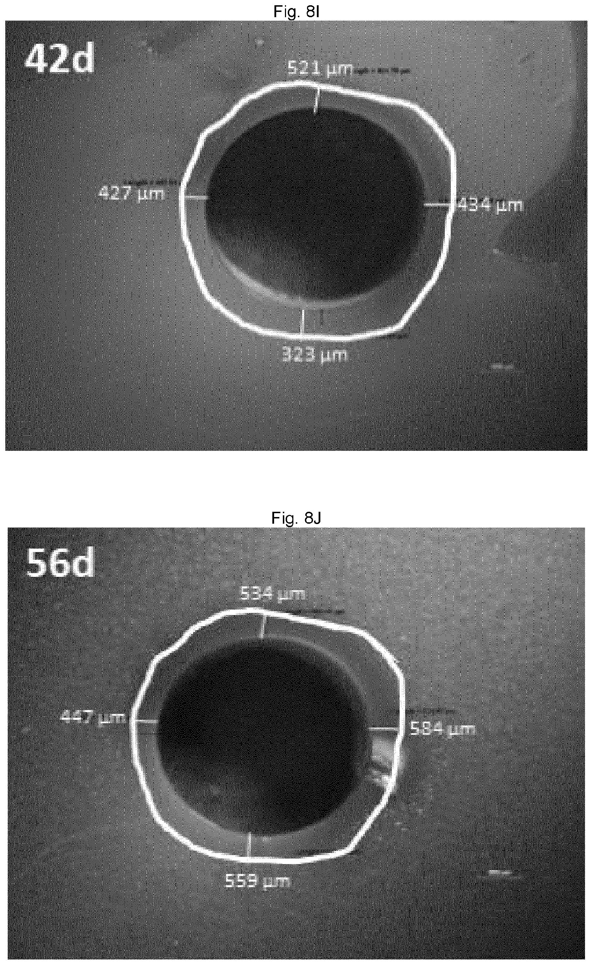

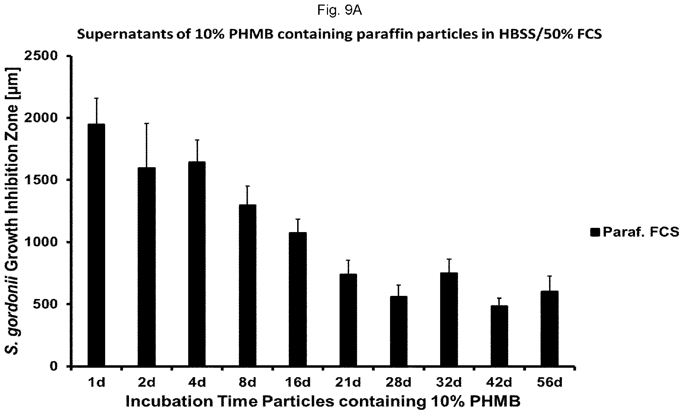

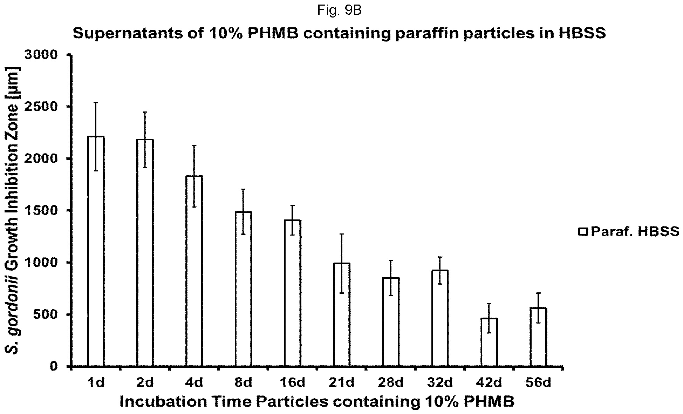

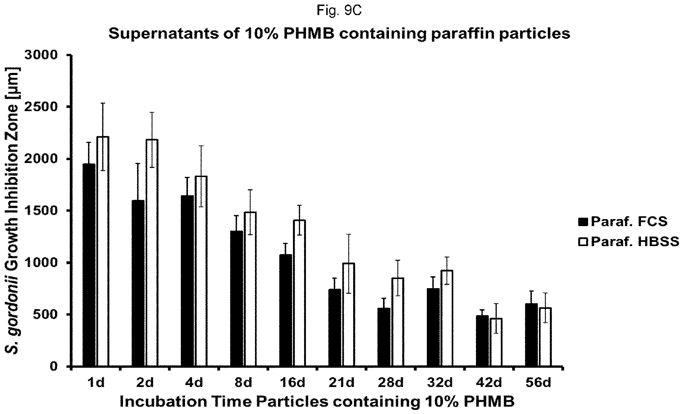

In another experiment the mean inhibition zone of the growth of *S*. *gordonii* using paraffin particles (Fig. 8A-J, Fig. 9A) containing 10% PHMB in HBSS with 50% FCS developed from 1942 µm after 24h to 1512 µm, 1425 µm, 1262 µm, 825 µm, 741 µm, 665 µm, 633 µm, 483 µm to 602 µm after 2 days (d), 4d, 8d, 16d, 21d, 28d, 32d, 42d and 56d. The mean inhibition using HBSS without serum (Fig. 9B) altered from 1948 µm after 24h to 1678 µm, 1299 µm, 1235 µm, 1259 µm, 1235 µm, 990 µm, 850 µm, 922 µm, 463 µm to 565 µm after 2d, 4d, 8d, 16d, 21d, 28d, 32d, 42d and 56d. Fig. 9C shows the development of the inhibition zones with/without serum in comparison over the time.

### Release kinetics by quantification using mass spectrometry:

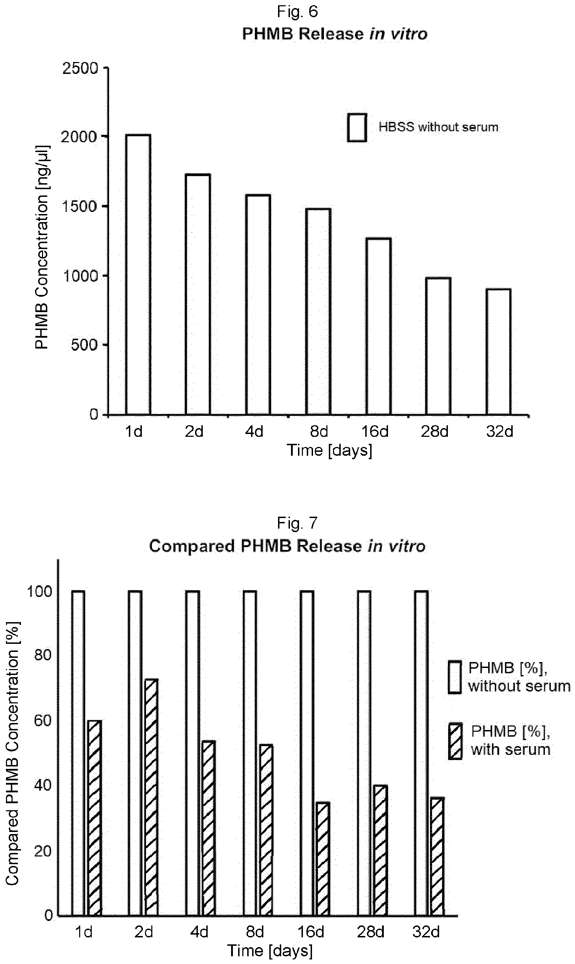

Samples without serum from the *in vitro* experiment (Fig. 6):
After 24h in 2µl sample volume 4027 ng (2014 ng/µl) PHMB are detected, 3456 ng (1728 ng/µl) after 48h, 3161 ng (1581 ng/µl) after 4d, 2961 ng (1481 ng/µl) after 8d, 2538 (1269 ng/µl) after 16d, 1970 ng (985 ng/µl) after 28d and 1806 ng (903 ng/µl) after 32d.

Samples with 50% serum (FCS) from the *in vitro* experiment (Fig. 7):
In comparison to the samples without serum after 24h in 2µl sample volume 60.03 % of the PHMB concentration is detected, 72.93 % after 48h, 53.86 % after 4d, 52.73 % after 8d, 34.92 % after 16d, 40.1 % after 28d and 34.42 % after 32d.

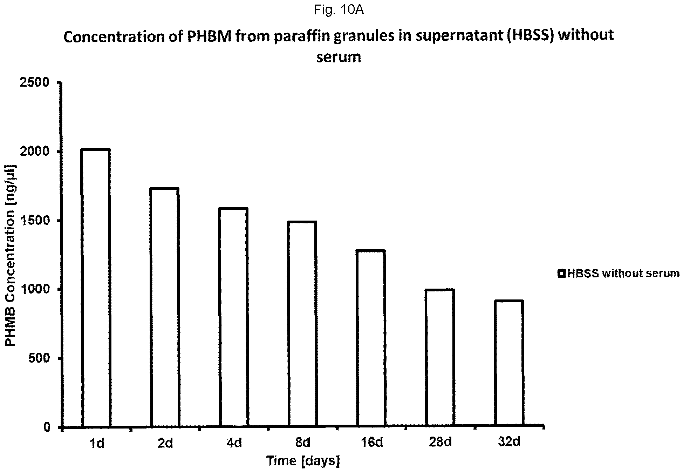

In another experiment (cf. samples without serum from the *in vitro* experiment, Fig. 10A) the following results are found:
After 24h in 2µl sample volume 4027 ng (2014 ng/µl) PHMB are detected, 3456 ng (1728 ng/µl) after 48h, 3161 ng (1581 ng/µl) after 4d, 2961 ng (1481 ng/µl) after 8d, 2538 (1269 ng/µl) after 16d, 1970 ng (985 ng/µl) after 28d and 1806 ng (903 ng/µl) after 32d.

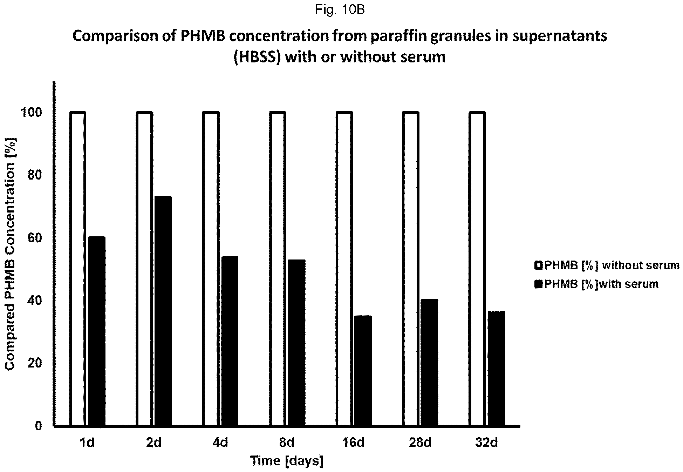

In another experiment (cf. samples with 50% serum (FCS) from the *in vitro* experiment, Fig. 10B) the following results are found:
In comparison to the samples without serum after 24h in 2µl sample volume 60.03 % of the PHMB concentration is detected, 72.93 % after 48h, 53.86 % after 4d, 52.73 % after 8d, 34.92 % after 16d, 40.1 % after 28d and 34.42 % after 32d.

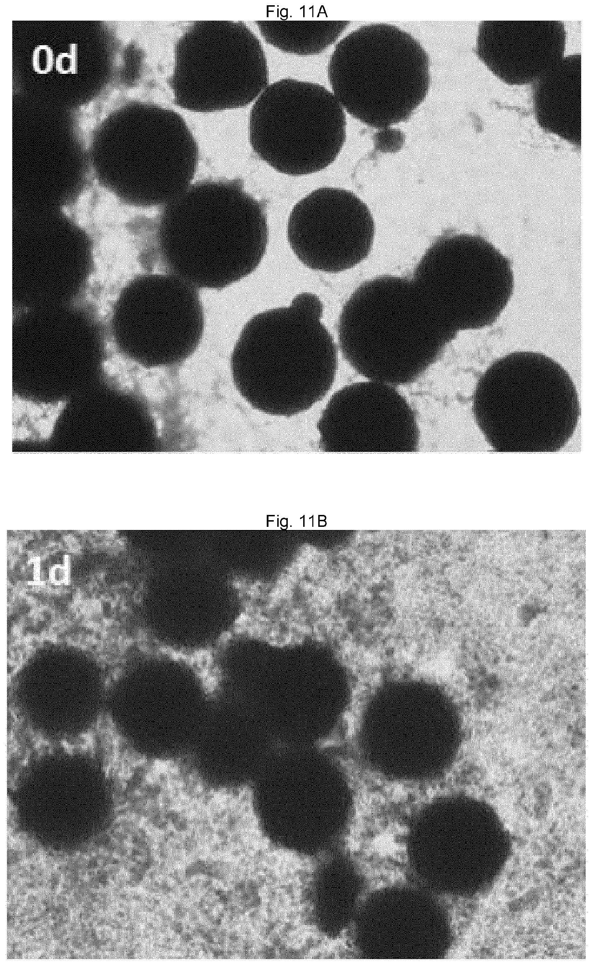

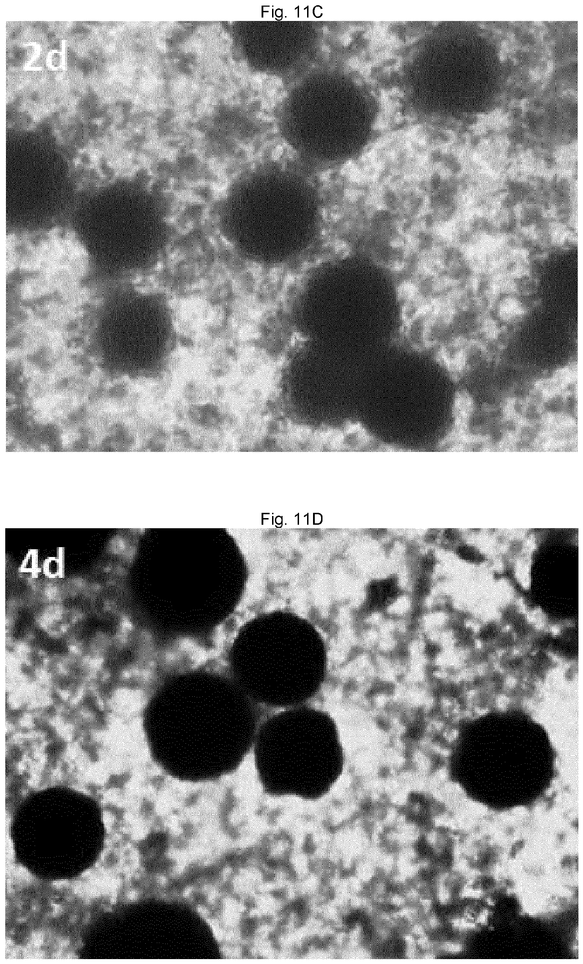

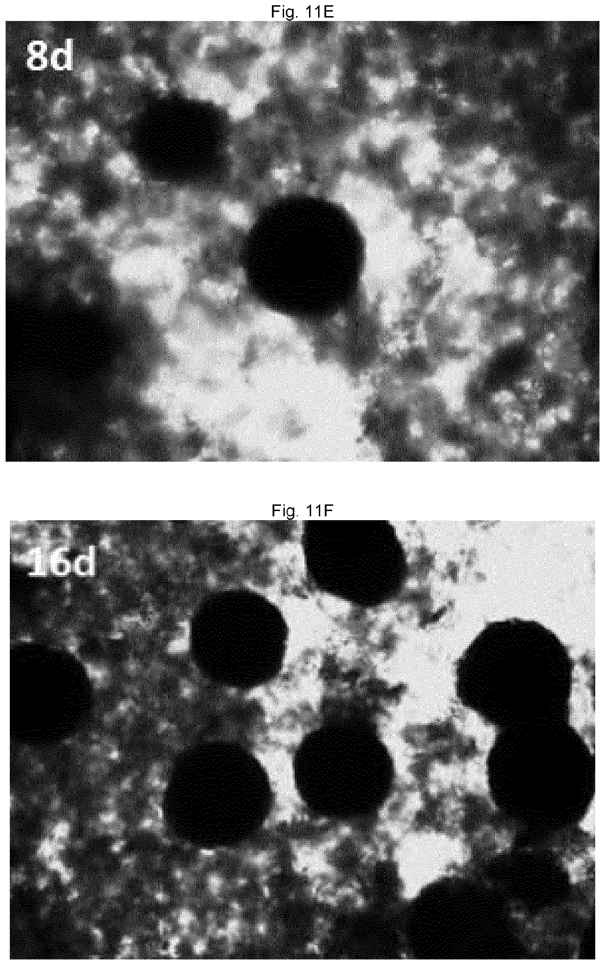

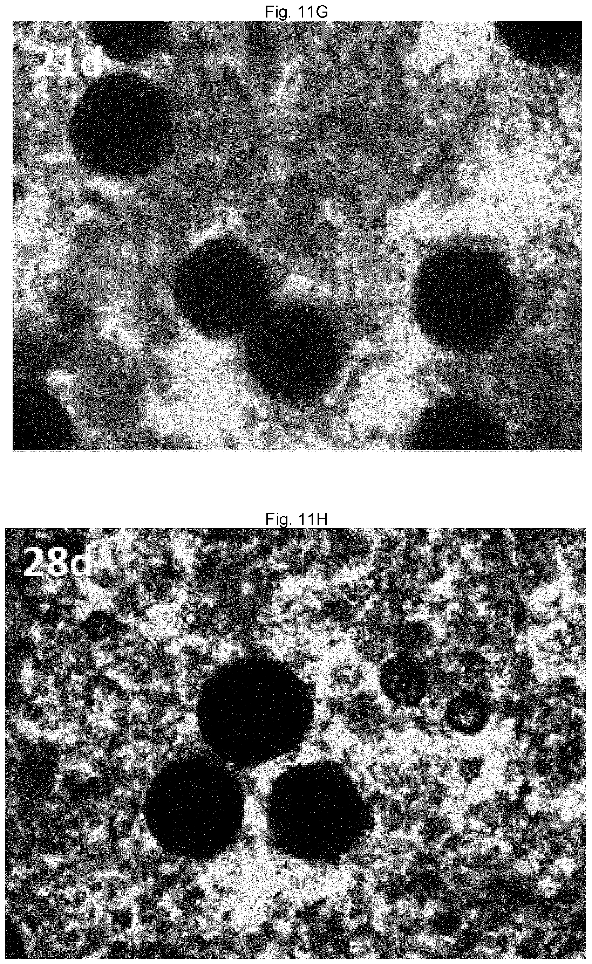

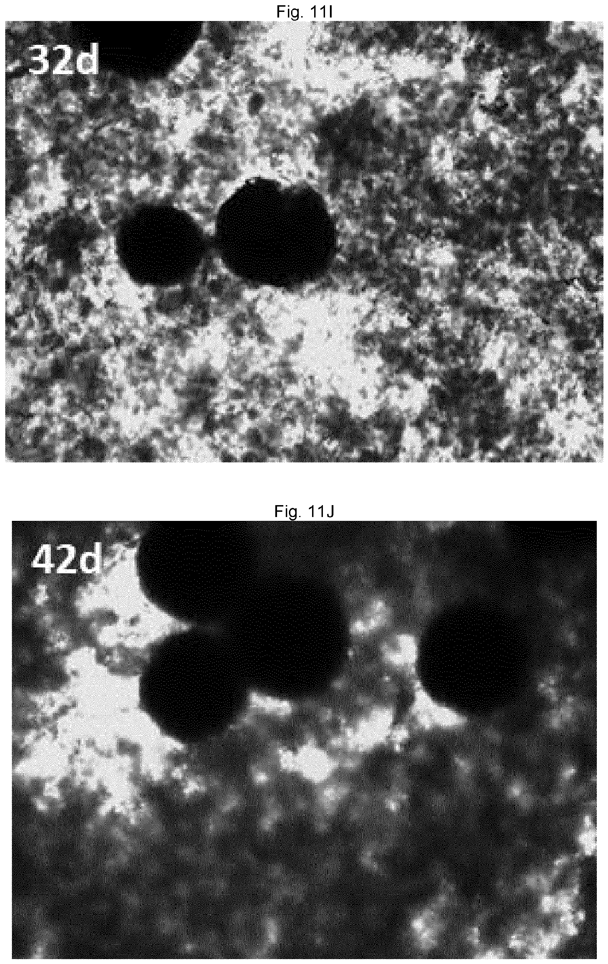

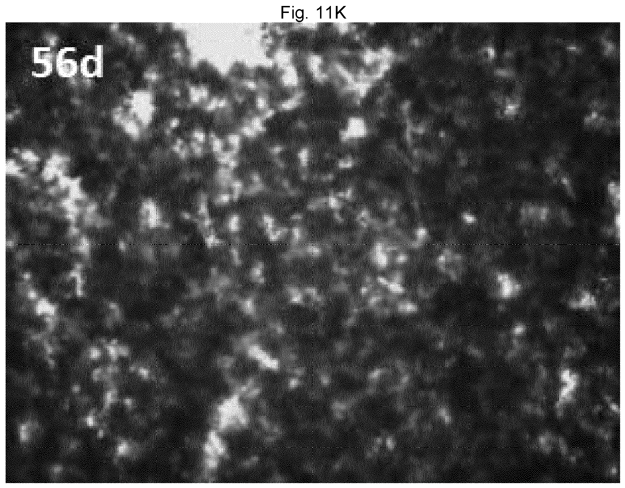

Melting behavior:
It was clearly demonstrated, that over the time the particles melted and passed over into the supernatant, more distinct in the FCS containing buffer (cf. Fig. 11).

The experiments described above show that the antibacterial efficacy of PHMB stays detectable over the whole time period of 56d. The release tests show that the PHMB is released from the beads in a constant manner. After the first 24h 1/5 of the calculated PHMB concentration (10 µg/µl) is detected (4.027 µg in 2 µl), this value being the base value in relation to which the long-time release kinetics is measured. The concentrations that are quantified in the samples by mass spectrometry from 48h, 4d, 8d, 16d, 28d and 32d are 86%, 79%, 74%, 63%, 49% and 45% of the 24h value (3.456 µg, 3.161 µg, 2.961 µg, 2.538 µg, 1.970 µg and 1.806 µg in 2 µl respectively). The presence of serum seems to inhibit the release or the detection of the PHMB.

### Description of the drawings

- Fig. 1A-G:: Exemplary images of *S*. *gordonii* growth inhibition zones caused by PHMB, released from the paraffin granules after the indicated incubation times (1d, 2d, etc.). The supernatants contain 50% fetal calf serum. The border of the inhibition zones is marked within all figures by a thick white curved line, exemplarily indicated in Fig. 1A with the reference sign 100. The extension of the growth inhibition zones are indicated by measurements from the upper, lower, left and right surface of the paraffin granules until the borderline of the growth inhibition zone as is recognizable in the microscopic images, indicated by thin straight white lines. In Fig. 1C the last digit of the value of the width of the inhibition zone on the right side of the paraffin granule is difficult to be recognized, therefore it is given here also: 1484 µm. In Fig. 1E the last digit of the value of the width of the inhibition zone on the right side of the paraffin granule is difficult to be recognized, therefore it is given here also: 814 µm.
- Fig. 2A-D:: Exemplary presentation of paraffin particles comprising 10% PHMB, suspended in HBSS with 50% serum (FCS); exemplarily chosen microscopic pictures of the particles and graphical display of the alteration of the growth inhibition zone of *S*. *mitis,* being in contact with the particles. The borders of the growth inhibition zones caused by PHMB, released from the paraffin granules after the indicated incubation times (1d, 2d, etc.). are marked within all figures by a manually drawn thick white curved line, indicated with the reference sign 100. The extension of the growth inhibition zones are quantitatively indicated by measurements from the upper, lower, left and right surface of the paraffin granules until the borderline of the growth inhibition zone as is recognizable in the microscopic images, indicated by straight white lines.
- Fig. 3A-D:: Exemplary presentation of paraffin particles comprising 10% PHMB, suspended in HBSS without serum (FCS); exemplarily chosen microscopic pictures of the particles. The borders of the growth inhibition zones caused by PHMB, released from the paraffin granules after the indicated incubation times (1d, 2d, etc.). are marked within all figures by a manually drawn thick white curved line, indicated with the reference sign 100. The extension of the growth inhibition zones are quantitatively indicated by measurements from the upper, lower, left and right surface of the paraffin granules until the borderline of the growth inhibition zone as is recognizable in the microscopic images, indicated by straight white lines.
- Fig. 4A-D:: Exemplary presentation of paraffin particles comprising 10% PHMB, suspended in HBSS without serum (FCS); exemplarily chosen microscopic pictures of the particles and graphical display of the alteration of the growth inhibition zone of *S*. *mitis*, being in contact with the particles. The borders of the growth inhibition zones caused by PHMB, released from the paraffin granules after the indicated incubation times (z. B. 1d, 2d, etc.) are marked within all figures by a manually drawn thick white curved line, indicated with the reference sign 100, partly interrupted. The extension of the growth inhibition zones are quantitatively indicated by measurements from the upper, lower, left and right surface of the paraffin granules until the borderline of the growth inhibition zone as is recognizable in the microscopic images, indicated by straight white lines.
- Fig. 5:: Exemplary comparative presentation of the development of the growth inhibition zone of *S*. *mitis* over time, being cultured in the presence of particles comprising 10% PHMB, with or without serum in the culture liquid.
- Fig. 6:: Exemplary presentation of results investigating the PHMB release from particles according to the invention in vitro (without serum (FCS)).
- Fig. 7:: Exemplary presentation of results investigating the PHMB release from particles according to the invention in vitro (with and without serum (FCS)).
- Fig. 8A-J:: Exemplary images of *S*. *gordonii* growth inhibition zones caused by release of PHMB from paraffin granules according to the invention. The paraffin granules released PHMB in supernatant (Hanks balanced salt solution = HBSS, HBSS contained 50% calf serum) after 1d, 2d, 4d, 8d, 16d, 21 d, 28d, 32d, 42d and 56 d incubation times (days = d, noted in the Figs. in the upper left corners).
- Fig. 9A-C:: Exemplary images of growth inhibition zones of *S*. *gordonii* on blood agar plates by PHMB. PHMB is released from paraffin granules in supernatant (Hanks balanced salt solution = HBSS) after 1d, 2d, 4d, 8d, 16d, 21 d, 28d, 32d, 42 d and 56d incubation times (days = d, noted in the Figs. in the upper left corners). Fig. 9A = supernatant with 50% serum, Fig. 9B = supernatant without serum, Fig. 9C = comparison between both (Fig. 9A and Fig. 9B), n = 9.
- Fig. 10A-B:: Exemplary concentration of PHMB released from paraffin granules in supernatants (HBSS) after the indicated incubation times. (Hanks balanced salt solution = HBSS) after 1d, 2d, 4d, 8d, 16d, 28d, 32d = incubation times (days = d). Fig. 10A = Supernatant with 50% serum, Fig. 10B = comparison of relative concentration between without and with 50% serum.
- Fig. 11A-K:: Melting behavior of 10% PHMB containing paraffin particles over a time of 8 weeks, each single figure indicating typical appearances at a certain time of the long-time experiment (Fig. 11A = initial time point, 0d; Fig. 11B = 1d; Fig. 11C = 2d etc., the individual time point being indicated at the upper left corner of each figure).

## Claims

1. A 1 to 60-day long-acting microsphere comprising polyhexamethylenebiguanidehydrochloride or its salt as a drug and at least one matrix material, independently from each other chosen from the list of matrix materials comprising paraffin wax, glycerin-monostearate, water, lecithin, glycerin, alginate, wherein
(i) the microsphere comprises
- polyhexamethylenebiguanidehydrochloride or its salt and
- an amount of paraffin wax per percent by weight of the sum of the components is
from 40 % by weight to 70 % by weight and/or
- an amount of glycerin-monostearate per percent by weight of the sum of the components is from 5 % by weight to 30 % by weight and/or
- an amount of water per percent by weight of the sum of the components is
from 50 % by weight to 30 % by weight and/or
- an amount of lecithin per percent by weight of the sum of the components is
from 0,5 % by weight to 20 % by weight,
- an amount of glycerin per percent by weight of the sum of the components which is within the range from 50 % by weight to 90 % by weight,
- an amount of alginate per percent by weight of the sum of the components which is within the range from 30 % by weight to 50 % by weight,
(ii) the microsphere has an average particle size of from 10 to 500 µm;
(iii) and the microsphere is satisfying at least one of conditions (1) to (2) below:
(1) a remaining ratio of the drug after 8 days in a release test is at least 70 % of the concentration detected after 1 day in the release test;.
(2) a remaining ratio of the drug after 32 days in a release test is at least 40 % of the concentration detected after 1 day in the release test.

2. The microsphere according to claim 1, **characterised in that** the microsphere has a core-shell-structure whereat
- the core of the microsphere comprises
- polyhexamethylenebiguanidehydrochloride or its salt and
- an amount of paraffin wax per percent by weight of the sum of the components of the core is from 50 % by weight to 60 % by weight and/or
- an amount of glycerin-monostearate per percent by weight of the sum of the components of the core is from 10 % by weight to 20 % by weight and/or
- an amount of water per percent by weight of the sum of the components of the core is from 10 % by weight to 20 % by weight and/or
- an amount of lecithin per percent by weight of the sum of the components of the core is from 1 % by weight to 10 % by weight
- the shell of the microsphere comprises
- an amount of glycerin per percent by weight of the sum of the components of the shell which is within the range from 60 % by weight to 80 % by weight and
an amount of alginate per percent by weight of the sum of the components of the shell which is within the range from 20 % by weight to 40 % by weight.

3. The microsphere according to claim 1, further comprising at least one pharmaceutically active substance, independently from each other chosen from the list comprising sodium ascorbate, selenium, zinc, vitamin D, vitamin E, vitamin K, vitamin K2, coenzyme Q10.

4. The microsphere according to claim 2, further comprising at least one pharmaceutically active substance, independently from each other chosen from the list comprising sodium ascorbate, selenium, zinc, vitamin D, vitamin E, vitamin K, vitamin K2, coenzyme Q10, wherein the at least one more pharmaceutically active substance is located either
- in the core and the shell of the microsphere or
- only in the core of the microsphere or
- only in the shell of the microsphere.

5. The microsphere according to any one of the preceeding claims, wherein the average size of the microsphere is not greater than 300 µm.

6. An agent comprising the microsphere of any one of the preceeding claims or a multitude of microspheres according to any one of the preceeding claims for use in preventing and/or treating wounds and/or acute or chronical inflammations on mammals,

7. The agent according to claim 6, **characterized in that** the microsphere or the multitude of microspheres is embedded in a gel comprising hyaluronic acid within the range from 0.001 % by weight to 10 % by weight and sterile water, whereat the hyaluronic acid comprises hyaluronic acid molecules with a molecular weight below 403 kDa.

8. The agent according to claim 6 or claim 7, **characterized in that** the agent is sterile, whereat the sterility is achieved by applying heat, uv-radiation or addition of an antimicrobial compound to the agent during manufacturing

9. The agent according to any one of the preceeding claims 6 - 8 for use in preventing and/or treating wounds and/or infections in mammals, being located at abscess-cavities and/or fistula cavities and/or gingival cavities and/or peri-implant niches.

10. The agent according to any one of the preceeding claims 6 - 8 for use in preventing and/or treating peri-implant inflammation.

11. The agent according to any one of the preceeding claims 6 - 8 for use in preventing and/or treating hidden infections fistulae, fissures and in regions adjacent to the root of tongue or the tonsils.

12. The agent according to any one of the preceeding claims 6 - 8 for use in preventing and/or treating
sinusitis.

13. The agent according to any one of the preceeding claims 6 - 8 for use in preventing and/or treating acute and/or chronic inflammations of niches of mucous membranes.

14. The agent according to any one of the preceeding claims 6 - 8 for use in preventing and/or treating acute and/or chronic inflammations of implant pockets.
